# EUROPEAN PATENT APPLICATION

(11) **EP 4 484 576 A1**
(43) Date of publication of application: **01.01.2025**
(21) Application number: 22927489.9
(22) Date of filing: 28.12.2022
(51) Int. Cl.: C12Q 1/6886

(54) **COMPOSITION FOR PREDICTING RESPONSE OF CANCER PATIENT TO IMMUNE CHECKPOINT INHIBITOR**

(30) Priority: 21.02.2022 KR 20220022574
(71) Applicant: Samsung Life Public Welfare Foundation, Seoul 04348 (KR)
(72) Inventor: KIM, Kyoung Mee, Seoul 06351 (KR); KANG, So Young, Seoul 06351 (KR)
(74) Representative: Ostertag & Partner Patentanwälte mbB
(86) International application number: PCT/KR2022/021530
(87) International publication number: WO 2023/158093

(57) **Abstract**

One aspect relates to a composition for predicting the response of a cancer patient to an immune checkpoint inhibitor and a method for predicting the same. According to one aspect, it has been confirmed that RT-qPCR results for CD274 mRNA in cancer cells of cancer patients treated with an immune checkpoint inhibitor have a significant correlation with PD-L1 immunohistochemistry (IHC) results and are superior to PD-L1 IHC results in terms of sensitivity, specificity, positive predictive value, negative predictive value, and accuracy. Accordingly, the composition for predicting the response of a cancer patient to an immune checkpoint inhibitor, according to one aspect, is superior to PD-L1 IHC in terms of the ability to predict the response to an immune checkpoint inhibitor in a cancer patient (in particular, a patient with gastric cancer or urothelial carcinoma), and the composition, by measuring the expression level of CD274 mRNA by means of RT-qPCR, has advantages of reducing the time needed to predict the response to an immune checkpoint inhibitor and enabling cancer diagnosis at an early stage.

## Description

### [Technical Field]

The present invention relates to a composition for predicting the response of a cancer patient to an immune checkpoint inhibitor.

### [Background Art]

Programmed death-ligand 1 (PD-L1) immunohistochemistry (IHC) is widely used to predict therapeutic response to immune checkpoint blockade (ICB). While these tests measure PD-L1 protein levels, they differ by antibody clone, staining platform, and interpretation methods. For instance, while assessment of PD-L1 expression in advanced gastric or gastroesophageal junction adenocarcinoma is performed using the PD-L1 IHC 22C3 pharmDx assay and a combined positive score (CPS), testing metastatic non-small cell lung cancer (NSCLC) samples relies on a tumor proportion score (TPS) instead of CPS. The Ventana SP142 assay is used in patients with urothelial carcinoma and to count immune cells (IC) within the tumor microenvironment. This variability in scoring methods has contributed to confounding results across clinical trials and in clinical practice, leading to uncertainty regarding the universal value of PD-L1 expression levels as biomarkers across tumor types. Furthermore, the use of formalin-fixed, paraffin-embedded (FFPE) archival tumor tissues prepared using nonstandardized fixation and storage methods may not provide predictable and intended results for adequate PD-L1 antigen retrieval, potentially increasing the heterogeneity of IHC intensity, extent, and topography of staining. All these factors complicate the use of PD-L1 levels as assessed by IHC to predict clinical response to ICB.

RNA-based assays on FFPE tissues are currently used in clinical settings to classify or predict the recurrence risk in patients affected by various tumor types. RNA-based gene expression assays include microarray, real-time quantitative reverse transcription polymerase chain reaction (RT-qPCR)-based assays, and RNA sequencing. The RT-qPCR assays are one of the most widely used methods of gene quantitation because they have a large dynamic range, high sensitivity, high specificity, and little to no post-amplification processing, and can increase the quantity of the sample throughput. In addition, the use of highly specific primers that target stably expressed genes provides a high level of specificity and sensitivity, allowing for the simultaneous measurement of several targets, including genes, for sample quality control purposes. Gene expression profiling by RT-qPCR has minimal input requirements and has the potential to be far more cost-effective than IHC methods. Furthermore, strong concordance between RT-qPCR and IHC has demonstrated the analytical validity of RT-qPCR, even for challenging FFPE tumor samples. Therefore, the implementation of gene-specific reverse transcription may considerably increase the success rate of the validation of molecular classifiers in FFPE sample cohorts.

In this study, the present inventors aimed to develop RT-qPCR to measure *CD274* mRNA expression levels correlating well to PD-L1 IHC that can help reduce testing time and save archival tumor tissues needed to conduct different IHC assays in the same patient. For this purpose, three RT-qPCR primers were designed and the results were compared with those of PD-L1 IHC, and the results were clinically validated in patients with gastric cancer (GC) and urothelial carcinoma treated with ICI.

### [Disclosure]

### [Technical Problem]

The present invention is directed to providing a composition for predicting the response of a cancer patient to an immune checkpoint inhibitor, including an agent capable of measuring the expression level of a CD274 gene in a sample isolated from a cancer patient.

The present invention is also directed to providing a kit for predicting the response of a cancer patient to an immune checkpoint inhibitor, including the composition.

The present invention is also directed to providing a method for predicting the response of a cancer patient to an immune checkpoint inhibitor, the method including: amplifying a CD274 gene in a sample isolated from a cancer patient; and
measuring the expression level of the CD274 gene.

The present invention is also directed to providing a use of an agent capable of measuring the expression level of a CD274 gene in a sample isolated from a cancer patient for predicting the response of a cancer patient to an immune checkpoint inhibitor.

### [Technical Solution]

One aspect of the present invention provides a composition for predicting the response of a cancer patient to an immune checkpoint inhibitor, including an agent capable of measuring the expression level of a CD274 gene in a sample isolated from a cancer patient.

The term "cancer patient" may refer to an individual who can have cancer as a disease, and specifically, the "individual" may be a mammal, such as a mouse, rat, cat, guinea pig, hamster, dog, monkey, chimpanzee, or human, and may be specifically a human.

The sample may be one or more selected from the group consisting of blood, plasma, serum, tissue, cells, lymphatic fluid, bone marrow fluid, saliva, ocular fluid, semen, brain extract, spinal fluid, synovial fluid, thymic fluid, ascitic fluid, amniotic fluid, cell tissue fluid, and cell culture fluid, and the sample may be specifically one or more selected from the group consisting of blood, plasma, serum, tissue, cells, lymphatic fluid, bone marrow fluid, cell tissue fluid, and cell culture fluid, and more specifically one or more selected from the group consisting of tissue, cells, cell tissue fluid, and cell culture fluid.

The term "gene" may refer to any nucleic acid sequence or part thereof that has a functional role in the encoding or transcription of a protein or in the regulation of the expression of other genes. A gene may consist of any nucleic acid encoding a functional protein or only a portion of a nucleic acid encoding or expressing a protein. A nucleic acid sequence may contain genetic abnormalities in exons, introns, initiation or termination regions, promoter sequences, other regulatory sequences, or unique sequences adjacent to the gene.

In one aspect, in the measurement of the expression level of the *CD274* gene, the amount of PD-L1 protein (the protein encoded by the *CD274* gene) or mRNA may be measured, and specifically, the amount of *CD274* mRNA may be measured.

When the amount of PD-L1 protein is measured in the measurement of the expression level of the *CD274* gene, "measurement of the amount of the protein" refers to a process of confirming the presence or absence and the expression level of the protein encoded by the gene in a subject sample. Specifically, the amount of the protein may be measured by one or more techniques selected from the group consisting of western blot, enzyme-linked immunosorbent assay (ELISA), radioimmunoassay (RIA), radioimmunodiffusion, an Ouchterlony immunodiffusion method, rocket immunoelectrophoresis, tissue immunostaining, an immunoprecipitation assay, a complement fixation assay, fluorescence activated cell sorter (FACS), and a protein chip.

When the amount of *CD274* mRNA is measured in the measurement of the expression level of the *CD274,* "measurement of the amount of mRNA" refers to a process of confirming the presence or absence and the expression level of the mRNA of the gene in a subject sample. The measurement of the amount of mRNA may be performed by one or more techniques selected from the group consisting of reverse transcription polymerase reaction (RT-PCR), real-time reverse transcriptase polymerase reaction (real-time RT-PCR), quantitative polymerase chain reaction (qPCR), competitive reverse transcription polymerase chain reaction (competitive RT-PCR), real-time quantitative polymerase chain reaction (RT-qPCR), inverse polymerase chain reaction (inverse PCR), long and accurate polymerase chain reaction (long and accurate PCR), RNaseprotection assay (RPA), northern blotting, and a DNA chip, and the technique may be specifically reverse transcription polymerase reaction (RT-PCR), competitive reverse transcription polymerase reaction (competitive RT-PCR), real-time reverse transcriptase polymerase reaction (real-time RT-PCR), real-time quantitative polymerase chain reaction (RT-qPCR), and more specifically, RT-qPCR.

In one aspect, the agent may be one or more selected from the group consisting of a primer, a probe, an aptamer, an antibody, and a peptide, which can specifically bind to a base sequence containing the *CD274* gene or a protein encoded by the base sequence, specifically a primer or probe capable of specifically binding to a base sequence including the *CD274* gene, more specifically a primer capable of specifically binding to a base sequence including the *CD274* gene.

Further, in one aspect, the agent may measure the expression level of one or more sites selected from the group consisting of the exon 1 and exon 2 junctions, the exon 3 and exon 4 junctions, and the exon 5 and exon 6 junctions of the CD274 gene.

Specifically, the agent may be one or more probes selected from the group consisting of a CD274 TaqMan probe for the exon 1 and exon 2 junctions (assay ID; Hs01125296_m1), a CD274 TaqMan probe for the exon 3 and exon 4 junctions (assay ID; Hs00204257_m1), and a CD274 TaqMan probe for the exon 5 and exon 6 junctions (assay ID; Hs01125301_m1).

The term "primer" refers to a nucleic acid sequence that can form complementary base pairs with a template strand and serve as a starting point for template strand copying, and may be, for example, a nucleic acid sequence of 5 to 50 amino acids. The primer is usually synthesized, but may also be used on naturally occurring nucleic acids. The sequence of the primer does not necessarily have to be exactly the same as the sequence of the template, but it must be sufficiently complementary to hybridize with the template.

The term "probe" refers to a material capable of specifically binding to a target material to be detected in a sample, and refers to a material capable of specifically confirming the presence of a target material in a sample through the binding.

The term "aptamer" refers to a small single-stranded nucleic acid (DNA or RNA) fragment that has the characteristics of being able to bind with high affinity and specificity to various types of substances, from low molecular weight compounds to proteins, and may be, for example, a single-stranded nucleic acid fragment consisting of 10 to 60 nucleotides.

The term "antibody" refers to a substance that specifically binds to an antigen to cause an antigen-antibody reaction, and may be a chimeric antibody, a humanized antibody, a human antibody, a synthetic antibody and/or an affinity matured antibody.

The term "peptide" refers to a polymer consisting of two or more amino acids linked together through amide bonds (or peptide bonds).

The primer, aptamer or probe may be chemically synthesized using a phosphoramidite solid support method or other well-known methods. Such a nucleic acid sequence may also be modified using many means known in the art. Non-limiting examples of such modification include methylation, capping, substitution of natural nucleotides with one or more homologs, and modification between nucleotides, for example, modification into an uncharged linker such as methyl phosphonate, phosphotriester, phosphoroamidate, or carbamate or a charged linker such as phosphorothioate or phosphorodithioate.

The antibody or peptide may be naturally derived or may be wholly or partially synthetically produced, and may contain synthesized sequences.

In one aspect, the cancer may be one or more selected from the group consisting of lung cancer, gastric cancer, liver cancer, bone cancer, pancreatic cancer, skin cancer, head and neck cancer, skin melanoma, uterine cancer, ovarian cancer, colorectal cancer, breast cancer, uterine sarcoma, fallopian tube carcinoma, endometrial carcinoma, cervical carcinoma, vaginal carcinoma, vulvar carcinoma, esophageal cancer, laryngeal cancer, small intestine cancer, thyroid cancer, parathyroid cancer, soft tissue sarcoma, urethral cancer, penile cancer, prostate cancer, multiple myeloma, chronic or acute leukemia, childhood solid tumors, lymphoma, bladder cancer, kidney cancer, renal cell carcinoma, renal pelvic carcinoma, axial contraction tumors, brainstem glioma, and urothelial carcinoma, and may be specifically one or more selected from the group consisting of gastric cancer and urothelial carcinoma.

The term "cancer" refers to a class of diseases characterized by the development of abnormal cells that have the ability to proliferate uncontrollably and to invade and destroy normal body tissue.

The term "immune checkpoint inhibitor" refers to a compound, for example, a drug (including an antibody), which inhibits or blocks proteins expressed by cells of the immune system, for example, T cells and some types of cancer cells. The protein may inhibit the immune response and block T cells from killing cancer cells, and when the protein is blocked, the inhibition of the immune system is overcome and the T cells may kill cancer cells.

In one aspect, the immune checkpoint inhibitor may target one or more immune checkpoints selected from the group consisting of PD-1, CTLA4, KIR, 4-1BB/TNFRS9, TIM3, and LAG3, specifically, the immune checkpoint inhibitor may target one or more immune checkpoints selected from the group consisting of PD-1, CTLA4, and LAG3, and more specifically, the immune checkpoint inhibitor may target PD-1, that is, PD-1/PD-L1.

In one aspect, the immune checkpoint inhibitor may be one or more selected from the group consisting of nivolumab, pembrolizumab, pidilizumab, ifilimumab, atezolizumab, tremelimumab, avelumab, and durvalumab, and more specifically may be one or more selected from the group consisting of pembrolizumab, nivolumab, atezolizumab, and durvalumab.

The term "response" refers to a clinical or therapeutic benefit conferred to a patient at risk of developing or having a disease or disorder, for example, cancer, and the benefit includes one or more of the following: prolonged survival (including overall survival and progression-free survival); inducing an objective response (including complete or partial response); or amelioration of signs or symptoms of cancer.

"Objective response" refers to a measurable response, such as a complete response (CR) or a partial response (PR).

"Complete response" means disappearance of all signs of cancer (for example, disappearance of all target lesions) in response to treatment.

"Partial response" refers to a decrease in the size or extent of somatic cancer of one or more tumors or lesions in response to treatment.

The term "prediction" may refer to the determination of a particular outcome, such as a response to treatment in advance, through the identification of a characteristic, such as the expression level of a particular gene.

In one embodiment, as a result of performing a comparative analysis of PD-L1 immunohistochemistry (IHC) results and RT-qPCR results for CD274 mRNA in cancer cells of patients with gastric cancer or urothelial carcinoma, it was confirmed that there is a significant correlation between the PD-L1 immunohistochemistry results and the RT-qPCR results for CD274 mRNA (see Example 1).

Further, in one embodiment, as a result of performing a comparative analysis of PD-L1 immunohistochemistry (IHC) results and RT-qPCR results for CD274 mRNA in cancer cells of patients with gastric cancer or urothelial carcinoma treated with an anti-PD-1/PD-L1 agent pembrolizumab, nivolumab, atezolizumab, or durvalumab as an immune checkpoint inhibitor, it was confirmed that there is a significant correlation between the PD-L1 immunohistochemistry results and the RT-qPCR results for CD274 mRNA (see Example 2).

In addition, in one embodiment, it was confirmed that there is a significant correlation between the survival of patients with gastric cancer or urothelial carcinoma and the PD-L1 immunohistochemistry (IHC) results or RT-qPCR results for CD274 mRNA in cancer cells of patients with gastric cancer or urothelial carcinoma (see Example 3).

Furthermore, in one embodiment, it was confirmed that the RT-qPCR results for CD274 mRNA in cancer cells of patients with gastric cancer or urothelial carcinoma are superior to PD-L1 immunohistochemistry (IHC) results in terms of sensitivity, specificity, positive predictive value (PPV), negative predictive value (NPV), and accuracy (see Example 4).

As a result of the investigations in Examples 1 to 4 above, it was confirmed that the composition for predicting the response of a cancer patient to an immune checkpoint inhibitor, according to one aspect, is superior to PD-L1 IHC in terms of the ability to predict the response to an immune checkpoint inhibitor in a cancer patient (in particular, a patient with gastric cancer or urothelial carcinoma).

Another aspect provides a kit for predicting the response of a cancer patient to an immune checkpoint inhibitor, including the composition.

The terms "cancer patient," "sample," "agent," "cancer," "immune checkpoint inhibitor," and the like may fall within the above-described scopes.

The kit may include a primer and a probe for measuring the expression level of the gene whose expression increases or decreases according to the therapeutic response to an immune checkpoint inhibitor, or an antibody that selectively recognizes a marker, as well as one or more other component compositions, solutions, or devices suitable for the analytical method.

The kit for measuring the mRNA expression level of the gene may be a kit including essential elements for performing RT-PCR. The RT-PCR kit may include not only each primer specific to the gene, but also a test tube or another appropriate container, a reaction buffer, deoxynucleotides (dNTPs), enzymes such as Taq-polymerase and reverse transcriptase, DNase, an RNase inhibitor, DEPC-water, sterilized water, and the like.

Still another aspect provides a method for predicting the response of a cancer patient to an immune checkpoint inhibitor, the method including: amplifying a CD274 gene in a sample isolated from a cancer patient; and
measuring the expression level of the CD274 gene.

The terms "cancer patient," "sample," "cancer," "immune checkpoint inhibitor," and the like may fall within the above-described scopes.

In one aspect, the CD274 gene may be CD274 mRNA.

Further, in one aspect, the CD274 gene may include one or more sites selected from the group consisting of the exon 1 and exon 2 junctions, the exon 3 and exon 4 junctions, and the exon 5 and exon 6 junctions of the CD274 gene.

In the measurement of the expression level of the CD274 gene, the expression level of the CD274 gene may be measured using one or more probes selected from the group consisting of a CD274 TaqMan probe for the exon 1 and exon 2 junctions (assay ID; Hs01125296_m1), a CD274 TaqMan probe for the exon 3 and exon 4 junctions (assay ID; Hs00204257_m1), and a CD274 TaqMan probe for the exon 5 and exon 6 junctions (assay ID; Hs01125301_m1).

In one aspect, in the amplification, one or more amplification methods selected from the group consisting of reverse transcription polymerase reaction (RT-PCR), real-time reverse transcriptase polymerase chain reaction (real-time RT-PCR), quantitative polymerase chain reaction (qPCR), competitive reverse transcription polymerase chain reaction (competitive RT-PCR), real-time quantitative polymerase chain reaction (RT-qPCR), inverse polymerase chain reaction (inverse PCR), and long and accurate polymerase chain reaction (long and accurate PCR) may be used, specifically one amplification method selected from the group consisting of RT-PCR, RT-qPCR, and real-time PCR may be used, and more specifically RT-qPCR may be used.

According to the method according to one aspect, by measuring the expression level of CD274 mRNA, the time required to predict the response of the immune check inhibitor may be shortened, and cancer (particularly gastric cancer or urothelial carcinoma) may be diagnosed early.

Yet another aspect is to provide a use of an agent capable of measuring the expression level of a CD274 gene in a sample isolated from a cancer patient for predicting the response of a cancer patient to an immune checkpoint inhibitor.

The terms "cancer patient," "sample," "cancer," "immune checkpoint inhibitor," and the like may fall within the above-described scopes.

### [Advantageous Effects]

According to one aspect, it has been confirmed that RT-qPCR results for CD274 mRNA in cancer cells of cancer patients treated with an immune checkpoint inhibitor have a significant correlation with PD-L1 immunohistochemistry (IHC) results and are superior to PD-L1 IHC results in terms of sensitivity, specificity, positive predictive value, negative predictive value, and accuracy. Accordingly, the composition for predicting the response of a cancer patient to an immune checkpoint inhibitor, according to one aspect, is superior to PD-L1 IHC in terms of the ability to predict the response to an immune checkpoint inhibitor in a cancer patient (in particular, a patient with gastric cancer or urothelial carcinoma), and the composition, by measuring the expression level of CD274 mRNA by means of RT-qPCR, has advantages of reducing the time needed to predict the response to an immune checkpoint inhibitor and enabling cancer diagnosis at an early stage.

### [Description of Drawings]

FIG. 1 is a view showing a schematic view of CD274 and TaqMan probes spanning exon 1-2, exon 3-4, and exon 5-6 boundaries.
FIG. 2 is a set of views showing the outcome of PD-L1 immunohistochemical staining in gastric cancer and the outcome of using SP142 assay in urothelial carcinoma.
FIGS. 3A to 3C are views showing correlations between PD-L1 score and CD274 mRNA expression level in gastric cancer and urothelial carcinoma.
FIGS. 4A to 4C are views showing PD-L1 combined positive score cutoffs for gastric cancer and PD-L1 immune cell cutoff for urothelial carcinoma.
FIG. 5 is a set of views showing the response outcomes to anti-PD-1/PD-L1 inhibitors in gastric cancer and urothelial carcinoma.
FIG. 6 is a set of views showing the PD-L1 expression and mRNA expression outcomes of CD274 in gastric cancer patients and urothelial carcinoma patients.
FIG. 7 is a set of views showing the survival outcomes of urothelial carcinoma patients to RT-qPCR and PD-1/PD-L1 treatment in the patients.

### [Modes of the Invention]

Hereinafter, the present invention will be described in more detail through examples. However, these examples are provided only for exemplarily describing the present invention, and the scope of the present invention is not limited by these examples.

### Reference Examples

### Reference Example 1. Patients and data collection

Data of 100 patients with severe gastric cancer (GC) (n = 59) or urothelial carcinoma (n = 41) and treated with palliative chemotherapy (n = 100) and additional anti-programmed death 1 (PD-1)/PD-L1 immunotherapy (n = 49) at Samsung Medical Center between December 2016 and January 2020 were retrospectively collected. The median age was 61.0 years (33 to 81 years), and 30 patients (61.2%) were male. For 49 patients treated with immunotherapy, the treatment responses were assessed every 6 to 12 weeks according to the Immune Response Evaluation Criteria in Solid Tumors (iRECIST). Patients with at least 6 weeks of follow-up period were included. The primary clinical endpoint of the present invention was objective response rate (ORR), defined as complete response (CR) or partial response (PR). The patients with progressive disease (PD) or stable disease (SD) were classified as non-responders. Clinicopathological data were retrospectively extracted from electronic medical records. The study was performed in accordance with the Institutional Review Board guidelines (IRB No. 2018-09-041-001) for data analysis and investigational treatments, and consent from patients was also obtained.

### Reference Example 2. RNA extraction and RT-qPCR

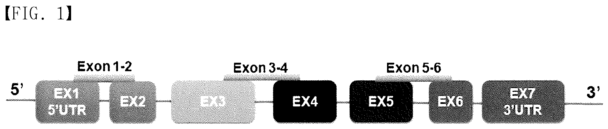

Total RNA was isolated from formalin-fixed, paraffin-embedded (FFPE) tumor tissues using the ReliaPrep^{™} FFPE Total RNA Miniprep System (Promega, WI, USA). Reverse transcription polymerase chain reaction (RT-PCR) was conducted using a highcapacity cDNA reverse transcription kit (Thermo Fisher Scientific, CA, USA) according to the manufacturer's instructions. Each target gene was analyzed using a gene expression assay containing forward and reverse primers and an FAM-labeled MGB TaqMan probe from Applied Biosystems (Thermo Fisher Scientific) according to the previous study of the present inventors. As a high correlation of programmed death-ligand 1 (PD-L1) immunohistochemistry (IHC) with NanoString nCounter assay was found, real-time quantitative reverse transcription polymerase chain reaction was conducted by selecting CD274 TaqMan probes spanning the exon 1-2 boundary (assay ID; Hs01125296_m1), exon 3-4 boundary (assay ID; Hs00204257_m1), and exon 5-6 boundary (assay ID; Hs01125301_m1) (FIG. 1). Polymerase chain reaction (PCR) amplifications were performed in triplicates using the following conditions on QuantStudio 6 (Thermo Fisher Scientific): 2 min at 50 °C and 10 min at 94 °C, followed by 40 cycles of 95 °C for 15 s and 60 °C for 60 s. The mRNA expression level of each gene was measured using the 2^{-ΔCt} (ΔCt = ΔCt_{target gene}-ΔCt_{GUSB2}) method.

### Reference Example 3. PD-L1 immunohistochemistry (IHC)

Gastric FFPE tissue blocks were cut into 4 µm sections and stained on an Autostainer Link 48 system (Agilent Technologies) using a Dako PD-L1 IHC 22C3 pharmDx kit (Agilent Technologies). For urothelial carcinoma, a rabbit anti-human PD-L1 monoclonal antibody (clone SP142; Ventana Medical Systems, Tucson, AZ, USA) was used as previously described. In GC, the combined positive score (CPS) of PD-L1 expression was calculated as the number of PD-L1-stained tumor and immune cells divided by the total number of viable tumor cells, multiplied by 100. The concordance rate between RT-qPCR and IHC was evaluated using CPS cutoffs of 1 and 10 for GC. In urothelial carcinomas, tumor-infiltrating immune cells (ICs) covering ≥ 5 of the tumor area were defined as PD-L1-positive.

### Reference Example 4. Statistical analyses of immunohistochemistry (IHC) and RT-qPCR

To compare IHC versus RT-qPCR, CPS ≥1 and ≥ 10 for GC, and IC ≥5 for urothelial carcinomas were used. To compute sensitivity, specificity, positive predictive value (PPV), negative predictive value (NPV), and accuracy, a positive result in IHC was considered as CPS ≥1 or ≥ 10 for GC, and IC ≥5 for urothelial carcinoma. Further, logistic regression was performed to evaluate the prediction of response based on tumor type, IHC results, and RT-qPCR results.

ORR (CR/PR) and disease control rate (DCR; CR/PR/SD) were compared with CD274 mRNA RT-qPCR results using two-tailed unpaired Student's t-tests. Receiver operating characteristic (ROC) curves were generated and the diagnostic value of different panels was assessed by computing the area under the ROC curve (AUC). Kaplan-Meier estimates of progression-free survival (PFS) and overall survival (OS) were compared using the log-rank test. All graphs were generated using GraphPad Prism version 9.0 (GraphPad Software Inc., San Diego, USA). Statistical significance was set at P < 0.05. All statistical analyses were performed using SPSS software version 27.0 (IBM Corp., Armonk, New York, USA).

### Examples

### Example 1. Analytical comparison of PD-L1 immunohistochemistry (IHC) and CD274 RT-qPCR results

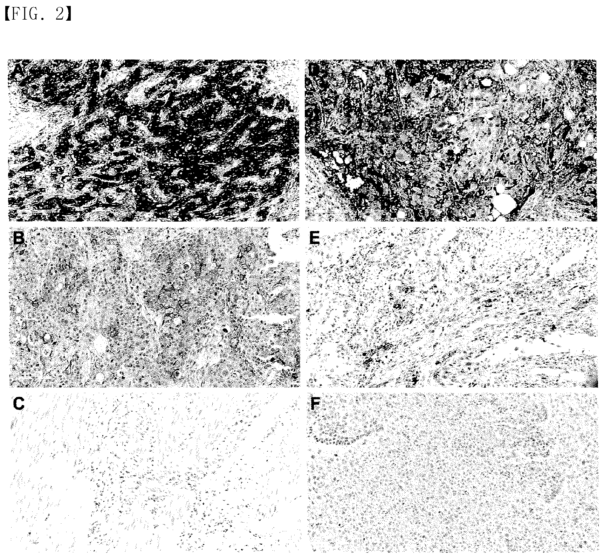

Using the 22C3 pharmDx assay, PD-L1 positivity with CPS ≥ 1 was found in 32 (54.2%) cases and ≥ 10 was identified in 13 (22 %) of 59 GC cases. The PD-L1 CPS ranged from 0 to 95, with a mean CPS of 9.24. Using the Ventana SP142 assay, PD-L1 positivity with IC ≥ 5 was identified in 12 (29.3 %) of 41 urothelial carcinomas. The PD-L1 IC in urothelial carcinomas ranged from 0 to 95, with a mean IC of 10.46. PD-L1 IHC in GC using 22C3 pharmDx and urothelial carcinomas using Ventana SP142 assays are shown (FIG. 2).

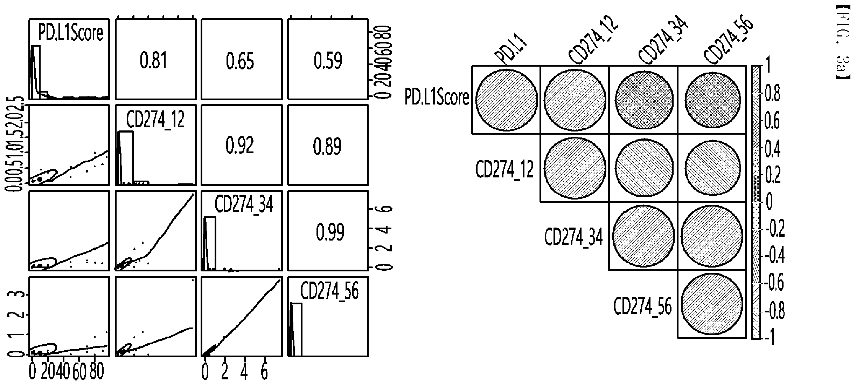

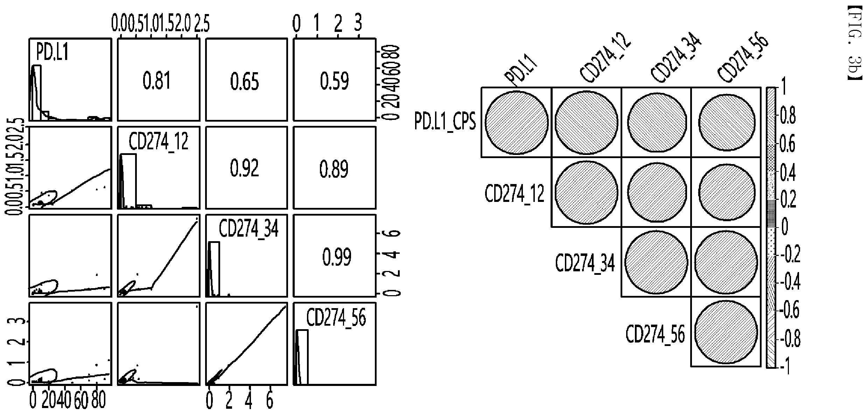

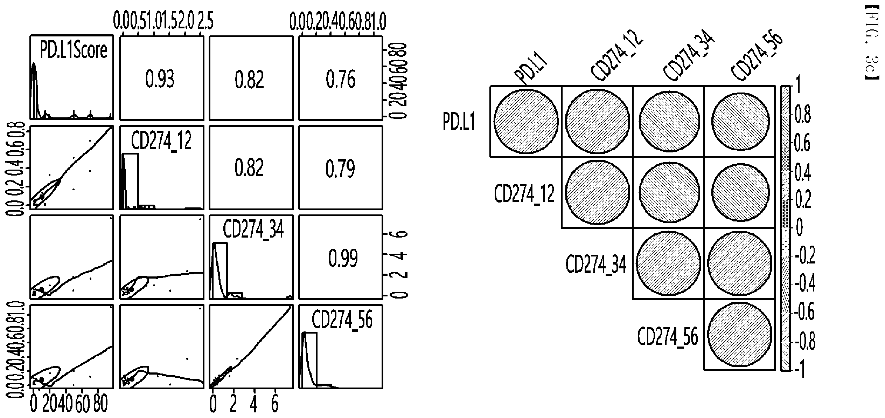

CD274 mRNA expression levels (relative quantification) spanning exon 1-2, exon 3-4, and exon 5-6 ranged from 0 to 2.4897 with a mean relative quantification (RQ) of 0. 1004, from 0 to 7.5214 with a mean RQ of 0.2371, and from 0 to 3.7064 with a mean RQ of 0.0928, respectively. CD274 mRNA expression levels within those three exonexon junctions showed a high correlation (Spearman correlation r²=0.92 for exon 1-2 and exon 3-4; r²=0.89 for exon 1-2 and exon 5-6; r²=0.99 for exon 3-4 and exon 5-6) (FIG. 3A). In all 100 samples evaluated, PD-L1 score was highly correlated with CD274 mRNA expression levels spanning exon 1-2 (r² = 0.81), exon 3-4 (r² = 0.65), and exon 5-6 (r² = 0.59) (FIG. 3A). In GC, PD-L1 CPS score with 22C3 pharmDx was significantly correlated with the exon 1-2 (r² = 0.81), exon 3-4 (r² = 0.67), and exon 5-6 (r² = 0.62) junctions of the CD274 (FIG. 3B). In urothelial carcinoma, Ventana SP142 PD-L1 IC score was significantly associated with exon 1-2 (r² = 0.93), exon 3-4(r² = 0.82), and exon 5-6 (r² = 0.76) junctions of CD274 (FIG. 3C).

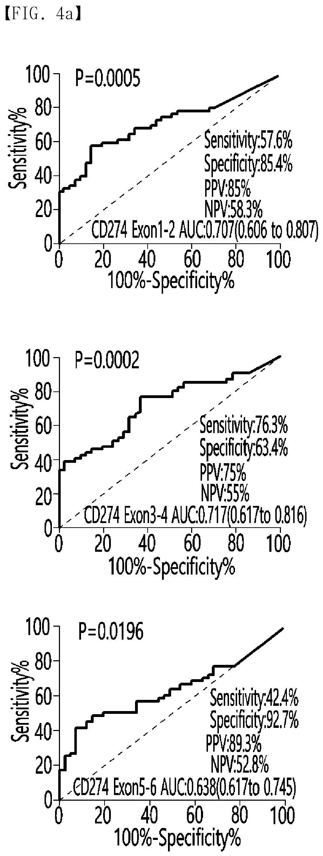

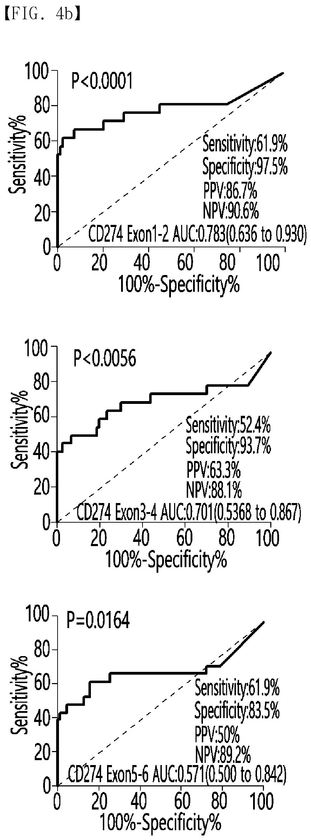

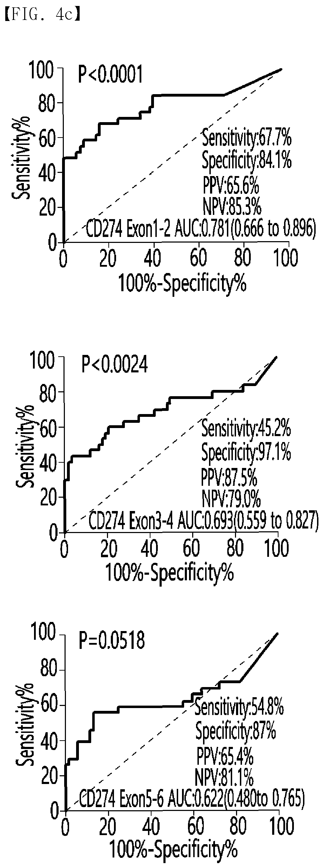

RQ cutoffs of CD274 mRNA expression in exon 1-2, exon 3-4, and exon 5-6 junctions were evaluated by AUC analyses based on PD-L1 CPS cutoffs of 1 and 10 for GC and PD-L1 IC cutoffs of 5 for urothelial carcinoma (Table 1 and FIGS. 4A to 4C). In GC, at a CPS cutoff of 10, the highest AUC value was 0.783, obtained from CD274 mRNA expression in exon 1-2 junction with a cutoff of 0.0722 (P < 0.0001). In urothelial carcinoma, AUC analysis based on PD-L1 IC cutoffs of IC 5 showed the highest AUC value of 0.781, obtained from CD274 mRNA expression in the exon 1-2 junction with a cutoff value of 0.0480 (P < 0.0001).

**[Table 1]**

| **Test Result** | **Test** | **Gastric** | **Urothelial** |
|---|---|---|---|
| ***Immunohistochemistry*** | | 22C3 pharmDx | SP142 |
| ≥1 CPS | IHC | 32 (54.2%) | |
| <1 CPS | IHC | 27 (45.8%) | |
| ≥10 CPS | IHC | 13 (22.0%) | |
| <10 CPS | IHC | 46 (78.0%) | |
| ≥5 IC | IHC | | 12 (29.3%) |
| <5 IC | IHC | | 29 (70.7%) |
| ***Cutoff Values of RT-qPCR*** | | | |
| ≥0.0276 RQ exon 1- exon 2 | CPS ≥1 | 22 (37.3%) | |
| <0.0276 RQ exon 1- exon 2 | | 37 (62.7%) | |
| ≥0.0563 RQ exon 3- exon 4 | | 34 (57.6%) | |
| <0.0563 RQ exon 3- exon 4 | | 25 (42.4%) | |
| 0.0511≥ RQ exon 5- exon 6 | | 14 (23.7%) | |
| <0.0511 RQ exon 5- exon 6 | | 45 (76.3%) | |
| ≥0.0722 RQ exon 1- exon 2 | CPS ≥ 10 | 13 (22.0%) | |
| <0.0722 RQ exon 1- exon 2 | | 46 (78.0%) | |
| ≥0.1966 RQ exon 3- exon 4 | | 9 (15.3%) | |
| <0.1966 RQ exon 3- exon 4 | | 50 (84.7%) | |
| ≥0.0528 RQ exon 5- exon 6 | | 13 (22.0%) | |
| <0.0528 RQ exon 5- exon 6 | | 46 (78.0%) | |
| ≥0.048 RQ exon 1- exon 2 | IC ≥ 5 | | 12 (29.3%) |
| <0.048 RQ exon 1- exon 2 | | | 29 (70.7%) |
| ≥0.1966 RQ exon 3- exon 4 | | | 7 (17.1%) |
| <0.1966 RQ exon 3- exon 4 | | | 34 (82.9%) |
| ≥0.0528 RQ exon 5- exon 6 | | | 13 (31.7%) |
| <0.0528 RQ exon 5- exon 6 | | | 28 (68.3%) |
| | **Total** | **59** | **41** |
| RT-qPCR, quantitative real-time polymerase chain reaction; IHC, immunohistochemistry; CPS, combined positive score | | | |

### Example 2. Prediction of patient response to anti-PD-1/PD-L1 inhibitor

Between May 2018 and October 2020, 49 patients were treated with anti PD-1/PD-L1 agents, and the treatment response was evaluated with a follow-up period of > 6 weeks (pembrolizumab [n = 16], nivolumab [n = 16], atezolizumab [n = 13], and durvalumab [n = 4]) (Table 2). The median number of cycles for PD-1/PD-L1 agents was 8.9 (range, 1-37) as of May 20, 2021, and the median follow-up duration was 11.3 months. Further, the clinicopathological characteristics of patients treated with anti-PD-1/PD-L1 therapy are summarized (Table 3).

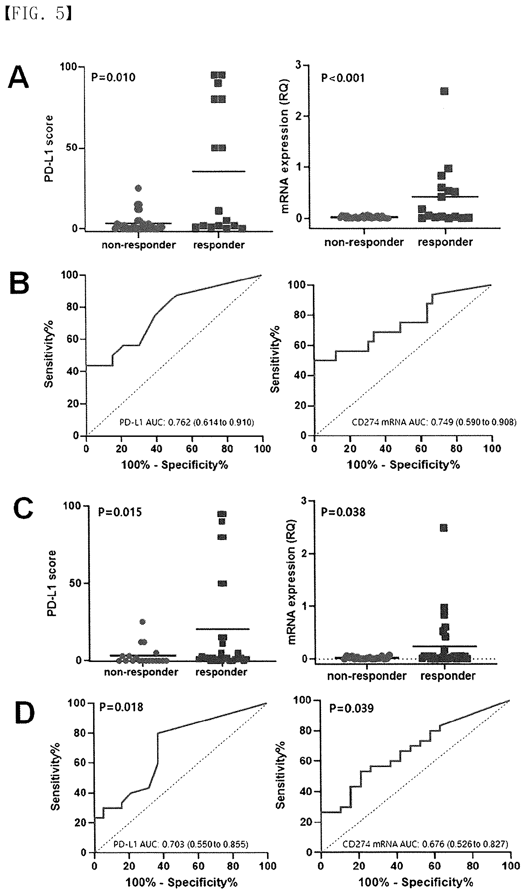

Anti-PD-1/PD-L1 responders (CR/PR, n= 16) and non-responders (PD/SD, n = 33) were identified using the iRECIST category of ORR. As a result, PD-L1 expression (P = 0.010) and CD274 high mRNA expression (P < 0.001) were significantly associated with the response to immunotherapy (FIG. 5A). ROC for predictive performance of PD-L1 IHC and mRNA expression of CD274 exon 1-2 showed high discriminatory ability. AUC and their 95% confidence intervals (CIs) were 0.76 (0.61 to 0.91) for PD-L1 and 0.75 (0.59 to 0.91) for mRNA expression of CD274 exon 1-2 (FIG. 5B). A similar association using the iRECIST category of DCR (CR/PR/SD, n = 30 and PD, n = 19) was observed for PD-L1 expression (P = 0.015) and CD274 high mRNA expression (P = 0.038) to predict responses to immunotherapy, with an AUC of 0.70 (0.55 to 0.86) and 0.68 (0.53 to 0.83), respectively (FIGS. 5C and 5D).

**[Table 2]**

| **Pat ien t No**. | **Tumo r type** | **Immune checkpoint inhibitor** | **Objecti ve respon se** | **CP S** | **IC** | **PD-L1 IHC antib ody clone** | **CD274 RQ exon 1-2, 0.0722 cut-off** | **CD27 4 RQ exon1 -2, 0.048 0 cutoff** | **PD-L1 IHC CPS 1% cutoff** | **PD-L1 IHC CPS 10% cutoff** | **PD-L1 IHC IC 5% cutoff** |
|---|---|---|---|---|---|---|---|---|---|---|---|
| 1 | GC | Pembrolizu mab | CR | 0 | | 22C3 | <0.072 2 | | <1% | <10 % | |
| 2 | GC | Pembrolizu mab | CR | 80 | | 22C3 | ≥0.072 2 | | <1% | ≥10 % | |
| 3 | GC | Nivolumab | PR | 2 | | 22C3 | <0.072 2 | | <1% | <10 % | |
| 4 | GC | Pembrolizu mab | PR | 1 | | 22C3 | <0.072 2 | | <1% | <10 % | |
| 5 | GC | Pembrolizu mab | PR | 80 | | 22C3 | ≥0.072 2 | | <1% | ≥10 % | |
| 6 | GC | Pembrolizu mab | PR | 90 | | 22C3 | ≥0.072 2 | | <1% | ≥10 % | |
| 7 | GC | Pembrolizu mab | PR | 95 | | 22C3 | ≥0.072 2 | | <1% | ≥10 % | |
| 8 | GC | Pembrolizu mab | PR | 2 | | 22C3 | <0.072 2 | | <1% | <10 % | |
| 9 | GC | Nivolumab | PR | 5 | | 22C3 | <0.072 2 | | <1% | <10 % | |
| 10 | GC | Pembrolizu mab | PR | 11 | | 22C3 | ≥0.072 2 | | <1% | ≥10 % | |
| 11 | GC | Durvalumab | SD | 1 | | 22C3 | <0.072 2 | | <1% | <10 % | |
| 12 | GC | Pembrolizu mab | SD | 0 | | 22C3 | <0.072 2 | | <1% | <10 % | |
| 13 | GC | Pembrolizu mab | SD | 0 | | 22C3 | <0.072 2 | | <1% | <10 % | |
| 14 | GC | Pembrolizu mab | SD | 3 | | 22C3 | <0.072 2 | | <1% | <10 % | |
| 15 | GC | Pembrolizu mab | SD | 5 | | 22C3 | <0.072 2 | | <1% | <10 % | |
| 16 | GC | Durvalumab | PD | 3 | | 22C3 | <0.072 2 | | <1% | <10 % | |
| 17 | GC | Durvalumab | PD | 0 | | 22C3 | <0.072 2 | | <1% | <10 % | |
| 18 | GC | Nivolumab | PD | 0 | | 22C3 | <0.072 2 | | <1% | <10 % | |
| 19 | GC | Nivolumab | PD | 0 | | 22C3 | <0.072 2 | | <1% | <10 % | |
| 20 | GC | Nivolumab | PD | 0 | | 22C3 | <0.072 2 | | <1% | <10 % | |
| 21 | GC | Nivolumab | PD | 0 | | 22C3 | <0.072 2 | | <1% | <10 % | |
| 22 | GC | Nivolumab | PD | 12 | | 22C3 | <0.072 2 | | <1% | ≥10 % | |
| 23 | GC | Nivolumab | PD | 0 | | 22C3 | <0.072 2 | | <1% | <10 % | |
| 24 | GC | Nivolumab | PD | 3 | | 22C3 | <0.072 2 | | <1% | <10 % | |
| 25 | GC | Pembrolizu mab | PD | 25 | | 22C3 | <0.072 2 | | <1% | ≥10 % | |
| 26 | GC | Pembrolizu mab | PD | 0 | | 22C3 | <0.072 2 | | <1% | <10 % | |
| 27 | GC | Pembrolizu mab | PD | 0 | | 22C3 | <0.072 2 | | <1% | <10 % | |
| 28 | GC | Pembrolizu mab | PD | 0 | | 22C3 | <0.072 2 | | <1% | <10 % | |
| 29 | GC | Nivolumab | PD | 0 | | 22C3 | <0.072 2 | | <1% | <10 % | |
| 30 | GC | Nivolumab | PD | 0 | | 22C3 | <0.072 2 | | <1% | <10 % | |
| 31 | GC | Nivolumab | PD | 0 | | 22C3 | <0.072 2 | | <1% | <10 % | |
| 32 | GC | Nivolumab | PD | 12 | | 22C3 | <0.072 2 | | <1% | ≥10 % | |
| 33 | GC | Durvalumab | PD | 2 | | 22C3 | <0.072 2 | | ≥1% | <10 % | |
| 34 | Uroth elial | Nivolumab | CR | | 0 | SP142 | 0.0115 3 | <0.04 80 | | | <5% |
| 35 | Uroth elial | Atezolizum ab | CR | | 50 | SP142 | 0.1734 2 | ≥0.04 80 | | | ≥5% |
| 36 | Uroth elial | Atezolizum ab | CR | | 50 | SP142 | 0.5122 8 | ≥0.04 80 | | | ≥5% |
| 37 | Uroth elial | Nivolumab | PR | | 1 | SP142 | 0.0524 1 | ≥0.04 80 | | | <5% |
| 38 | Uroth elial | Nivolumab | PR | | 2 | SP142 | 0.0282 2 | <0.04 80 | | | <5% |
| 39 | Uroth elial | Atezolizum ab | PR | | 95 | SP142 | 0.8328 4 | ≥0.04 80 | | | ≥5% |
| 40 | Uroth elial | Atezolizum ab | SD | | 0 | SP142 | 0.0000 0 | <0.04 80 | | | <5% |
| 41 | Uroth elial | Atezolizum ab | SD | | 0 | SP142 | 0.0153 5 | <0.04 80 | | | <5% |
| 42 | Uroth elial | Atezolizum ab | SD | | 1 | SP142 | 0.0203 8 | <0.04 80 | | | <5% |
| 43 | Uroth elial | Atezolizum ab | SD | | 1 | SP142 | 0.0448 7 | <0.04 80 | | | <5% |
| 44 | Uroth elial | Atezolizum ab | SD | | 1 | SP142 | 0.0511 2 | ≥0.04 80 | | | <5% |
| 45 | Uroth elial | Atezolizum ab | SD | | 2 | SP142 | 0.0000 0 | <0.04 80 | | | <5% |
| 46 | Uroth elial | *Atezolizuma b* | SD | | 2 | SP142 | 0.0124 5 | <0.04 80 | | | <5% |
| 47 | Uroth elial | Atezolizum ab | SD | | 15 | SP142 | 0.0000 0 | <0.04 80 | | | ≥5% |
| 48 | Uroth elial | Atezolizum ab | SD | | 15 | SP142 | 0.0174 2 | <0.04 80 | | | ≥5% |
| 49 | Uroth elial | Atezolizum ab | PD | | 5 | SP142 | 0.0000 0 | <0.04 80 | | | <5% |
| RQ, relative quantification; IHC, immunohistochemistry; GC, gastric cancer; CR, complete response; PR, partial response; SD, stable disease; PD, progressive disease; CPS, combined positive score; IC, immune cell | | | | | | | | | | | |

**[Table 3]**

| | | **Anti-PD-1/PD-L1 patients, No (%)** | **Overall response rate (CR/PR), (%)** | **P-value** | **Disease control rate (CR/PR/SD), (%)** | **P-value** |
|---|---|---|---|---|---|---|
| ***Overall*** | | **49** | **16 (32.7%)** | | **30 (61.2%)** | |
| Age | | | | 0.261 | | 0.043 |
| <65 | | 30 (61.2%) | 8 (26.7%) | | 15 (50%) | |
| ≥ 65 | | 19 (38.8%) | 8 (42.1%) | | 15 (78.9%) | |
| Sex | | | | 0.045 | | 0.001 |
| | Male | 30 (61.2%) | 13 (43.3%) | | 24 (80%) | |
| | Female | 19 (38.8%) | 3 (15.8%) | | 6 (31.6%) | |
| **Gastric cancer** | | **33** | 10 (30.3%) | | 15 (45.5%) | |
| | PD-L1 CPS cutoff 1 | 18 | 9 (50%) | 0.007 | 12 (66.7%) | 0.007 |
| | RT-qPCR cutoff 0.0276 | 15 | 6 (40%) | 0.269 | 10 (66.7%) | 0.025 |
| | PD-L1 CPS cutoff | 8 | 5 (62.5%) | 0.023 | 5 (62.5%) | 0.266 |
| 10 | | | | | | |
| | RT-qPCR cutoff 0.0722 | 5 | 5 (100%) | <0.001 | 5 (100%) | 0.008 |
| **Urothelial carcinoma** | | **16** | 6 (37.5%) | | 15 (93.8%) | |
| | PD-L1 IC cutoff 5 | 6 | 3 (50%) | 0.424 | 5 (83.3%) | 0.182 |
| | RT-qPCR cutoff 0.0480 | 5 | 4 (80%) | 0.018 | 5 (100%) | 0.486 |
| RT-qPCR, quantitative reverse transcription-polymerase chain reaction; CR, complete response; PR, partial response; SD, stable disease; PD, progressive disease; CPS, combined positive score | | | | | | |

### Example 3. Correlation of patient survival with PD-L1 immunohistochemistry (IHC) and CD274 RT-qPCR results

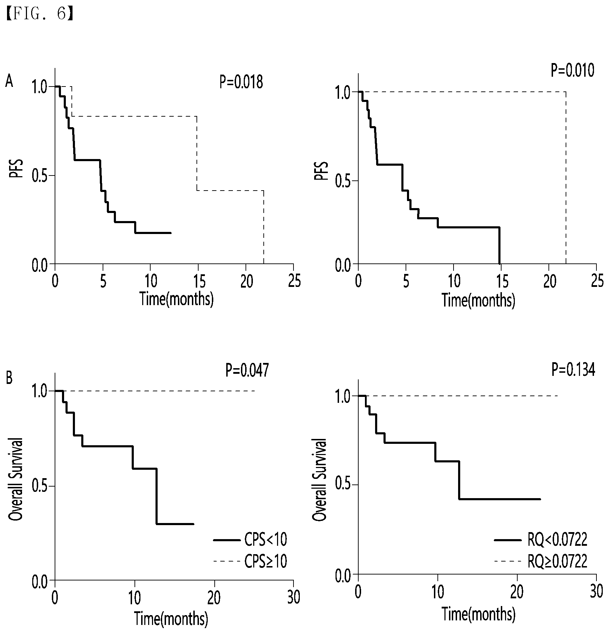

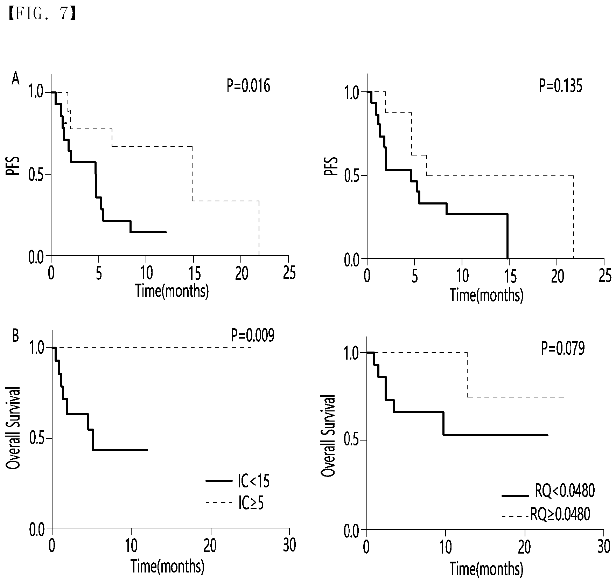

In GC patients, progression-free survival (PFS) was strongly associated with PD-L1 expressions (P = 0.018) and high mRNA expression of CD274 spanning exon 1-2 junction (P = 0.010) (FIG. 6A). Similar association was also observed between overall survival (OS) and PD-L1 expressions (P = 0.047), however, OS was not significantly associated with mRNA expressions (P = 0.134) (FIG. 6B). For patients with urothelial carcinoma, PD-L1 expression was significantly associated with both PFS (P = 0.016) and OS (P = 0.009). However, mRNA expression of CD274 exon 1-2 junction did not significantly correlate with PFS and OS of patients (FIG. 7).

### Example 4. Clinical utility of PD-L1 immunohistochemistry (IHC) versus CD274 RT-qPCR results

Standard parameters of sensitivity, specificity, PPV, NPV, and accuracy were used to compare the clinical utility of PD-L1 assessment with IHC and RT-qPCR (Table 4). To acquire the best performance to predict responses for immunotherapy, two values were used in GC samples (CPS ≥ 1% and 10%; RQ ≥ 0.0276 and ≥ 0.0772). PD-L1 CPS ≥ 1% showed the highest sensitivity (90%) and RT-qPCR with an average RQ cutoff of 0.0722 showed the highest specificity (100%) in GC. The highest sensitivity was achieved with CPS ≥1% in GC samples (90%) although PPV was very low (50%). In urothelial carcinomas, higher sensitivity (66.7%) and specificity (90%) were seen with a RT-qPCR with higher AUC values compared to PD-L1 IHC.

**[Table 4]**

| Prediction method | Sensitivity | Specificity | PPV | NPV | AUC on ROC (95% CI) |
|---|---|---|---|---|---|
| Gastric cancer IHC ≥ 1% | 90.0% | 60.9% | 50.0% | 93.3% | 0.75 (0.58-0.93) |
| Gastric cancer RQ ≥ 0.0276 | 60.0% | 60.9% | 40.0% | 77.8% | 0.60 (0.39-0.82) |
| Gastric cancer IHC ≥ 10% | 50.0% | 87.0% | 62.5% | 80.0% | 0.69 (0.47-0.90) |
| Gastric cancer RQ ≥ 0.0772 | 50.0% | 100.0% | 100.0% | 82.1% | 0.75 (0.54-0.96) |
| Urothelial carcinoma IHC ≥ 5% | 50.0% | 70.0% | 50.0% | 70.0% | 0.60 (0.30-0.90) |
| Urothelial carcinoma RQ ≥ 0.0480 | 66.7% | 90.0% | 80.0% | 81.8% | 0.78 (0.52-1.00) |
| RQ, relative quantification; IHC, immunohistochemistry; RT-qPCR, quantitative real-time polymerase chain reaction; AUC, area under curve; ROC, receiver operating characteristic | | | | | |

## Claims

1. A composition for predicting the response of a cancer patient to an immune checkpoint inhibitor, comprising an agent capable of measuring the expression level of a CD274 gene in a sample isolated from a cancer patient.

2. The composition of claim 1, wherein the sample is one or more selected from the group consisting of blood, plasma, serum, tissue, cells, lymphatic fluid, bone marrow fluid, saliva, ocular fluid, semen, brain extract, spinal fluid, synovial fluid, thymic fluid, ascitic fluid, amniotic fluid, cell tissue fluid, and cell culture fluid.

3. The composition of claim 1, wherein, in the measurement of the expression level of the CD274 gene, the amount of CD274 mRNA is measured.

4. The composition of claim 1, wherein the agent is a primer or probe capable of specifically binding to a base sequence of the *CD274* gene.

5. The composition of claim 1, wherein the agent measures the expression level of one or more sites selected from the group consisting of the exon 1 and exon 2 junctions, the exon 3 and exon 4 junctions, and the exon 5 and exon 6 junctions of the CD274 gene.

6. The composition of claim 1, wherein the cancer is one or more selected from the group consisting of lung cancer, gastric cancer, liver cancer, bone cancer, pancreatic cancer, skin cancer, head and neck cancer, skin melanoma, uterine cancer, ovarian cancer, colorectal cancer, breast cancer, uterine sarcoma, fallopian tube carcinoma, endometrial carcinoma, cervical carcinoma, vaginal carcinoma, vulvar carcinoma, esophageal cancer, laryngeal cancer, small intestine cancer, thyroid cancer, parathyroid cancer, soft tissue sarcoma, urethral cancer, penile cancer, prostate cancer, multiple myeloma, chronic or acute leukemia, childhood solid tumors, lymphoma, bladder cancer, kidney cancer, renal cell carcinoma, renal pelvic carcinoma, axial contraction tumors, brainstem glioma, and urothelial carcinoma.

7. The composition of claim 1, wherein the immune checkpoint inhibitor targets one or more immune checkpoints selected from the group consisting of PD-1, CTLA4, KIR, 4-1BB/TNFRS9, TIM3, and LAG3.

8. The composition of claim 1, wherein the immune checkpoint inhibitor is one or more selected from the group consisting of pembrolizumab, nivolumab, atezolizumab, and durvalumab.

9. A kit for predicting the response of a cancer patient to an immune checkpoint inhibitor, comprising the composition of claim 1.

10. A method for predicting the response of a cancer patient to an immune checkpoint inhibitor, the method comprising: amplifying a CD274 gene in a sample isolated from a cancer patient; and
measuring the expression level of the CD274 gene.

11. The method of claim 10, wherein the CD274 gene is CD274 mRNA.

12. The method of claim 10, wherein the CD274 gene comprises one or more sites selected from the group consisting of the exon 1 and exon 2 junctions, the exon 3 and exon 4 junctions, and the exon 5 and exon 6 junctions of the CD274 gene.

13. The method of claim 10, wherein real-time quantitative polymerase chain reaction (RT-qPCR) is used for the amplification.

14. A use of an agent capable of measuring the expression level of a CD274 gene in a sample isolated from a cancer patient for predicting the response of the cancer patient to an immune checkpoint inhibitor.
